# EUROPEAN PATENT SPECIFICATION

(11) **EP 2 988 703 B1**
(45) Date of publication and mention of the grant of the patent: **28.06.2017**
(21) Application number: 14732668.0
(22) Date of filing: 25.04.2014
(51) Int. Cl.: A61F 2/46

(54) **DELIVERY DEVICE FOR GRAFT MATERIAL**
FREISETZUNGSVORRICHTUNG FÜR TRANSPLANTATE
DISPOSITIF ADMINISTRATION DE GREFFON

(30) Priority: 26.04.2013 US 201313871743
(43) Date of publication of application: 02.03.2016
(73) Proprietor: Medtronic Xomed, Inc., Jacksonville, FL 32216 (US)
(72) Inventor: SHADECK, Louis M., Dunnellon, FL 34433 (US); BERMAN, Phillip J., Jacksonville, Florida 32257 (US)
(74) Representative: Hughes, Andrea Michelle
(86) International application number: PCT/US2014/035493
(87) International publication number: WO 2014/176526

(56) References cited:
- EP-A1- 2 436 342
- WO-A1-95/31948
- US-A1- 2009 318 925
- US-A1- 2010 211 058
- US-A1- 2012 265 167

## Description

### Background

Concepts presented herein relate to delivery of graft material to a target site. Example applications that utilize graft material include rhinologic functional endoscopic sinus surgery (FESS), spinal, orthopedic and arthroscopic procedures. Regardless of the exact procedure, various instruments for delivery of graft material are currently employed. In some procedures to delivery material to a target site, surrounding structures can be sensitive and thus contact between these structures and the instruments should be avoided. As such, current procedures can be overtly time consuming and require that the instruments pass sensitive structures multiple times to perform the delivery of material. This situation increases the chance of damage to sensitive structures adjacent the target site.

EP 2436342 A1 discloses a delivery device for delivering graft material to a target site, comprising a hand piece maintaining an actuator mechanism, a tube defining a lumen, an open end and a connection mechanism coupled to the hand piece, and a plunger positioned within the lumen of the tube and coupled to the actuator mechanism which is configured to move the plunger relative to the open end within the lumen from a loading position to an extended position.

### Summary

A method of delivering graft material to a surgical site includes positioning graft material in a tube of a delivery device. The tube defines an open end and a bend portion along its length. A plunger within the tube is advanced to dispense the graft material through the open end.

According to the present invention, a delivery device for delivering graft material to a target site includes the features of claim 1.

### Brief Description of the Drawings

Fig. 1 is a side view of a delivery device according to a first embodiment.
Fig. 2 is a side view of a delivery device according to a second embodiment.
Fig. 3 is a side view of a distal end of a delivery device.
Fig. 4 is a side view of an alternative plunger of a delivery device.
Figs. 5 and 6 are schematic views of steps in using a delivery tube to deliver graft material to a cavity.

### Detailed Description

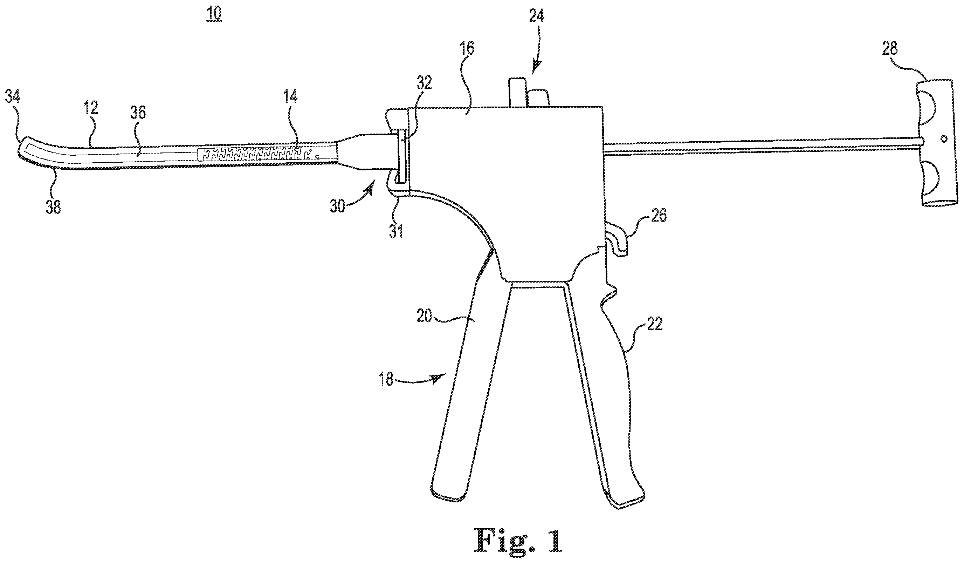

Fig. 1 is a side view of a first embodiment of a delivery device 10. Device 10 includes a delivery tube 12, a plunger 14 positioned within the delivery tube 12 and a hand piece 16 coupled with the tube 12 and plunger 14. Hand piece 16 maintains an actuator mechanism 18 configured to move plunger 14 with respect to tube 12. In one embodiment, the actuator mechanism 18 can move plunger 14 in a controlled, metered manner as desired. In the illustrated embodiment, actuator mechanism 18 includes a first handle 20, a second handle 22 and a ratcheting mechanism generally indicated at 24. As a user squeezes handles 20 and 22 together, ratcheting mechanism 24 moves to operate and advance plunger 14 along the tube 12. Advancement of the plunger 14 can be controlled to advance a predetermined distance each time the actuation mechanism 18 is actuated. This predetermined distance corresponds to a volume of material within the tube that will be dispersed. In a further embodiment, tube 12 can include markings to denote an amount of material positioned with the tube 12.

Hand piece 16 can also include a release mechanism 26 that releases plunger 14 from engagement with the ratchet mechanism 24. In particular, in order to move plunger 14 manually with respect to tube 12, a user can depress the release mechanism 26 and operate a handle 28 to move the plunger 14 to a desired position along tube 12.

Details of the tube 12 are discussed below. In general, however, tube 12 can include a suitable connection mechanism 30 that engages a corresponding receiving mechanism 31 on the hand piece 16 so as to secure the tube 12 to the hand piece 16 during operation of the device 10. Tube 12 includes a first, proximal end 32 and a distal, open end 34. The tube 12 further defines a lumen 36 configured to transport graft material therein. The tube 12 further includes a bend portion 38 positioned between the proximal end 32 and the proximal end 34 and the distal end 34. In one embodiment, a distance from the distal end 34 to the bend portion 38 is approximately in a range from 2,54 to 3,81 cm ( 1.0 to 1.5 inches). Furthermore, the bend portion 38 is defined as having approximately a 10,16 cm ( 4.0 inch) radius.

In any event, plunger 14 in Fig. 1 is illustrated in a retracted or loading position that allows graft material to be inserted through distal end 34 and into lumen 36. Once the material is loaded into lumen, plunger 14 can be advanced toward the distal end 34 (i.e., to an extended or delivery position) in order to advance material within the lumen 36 along the tube 12. Ultimately, the material exits the distal end 34.

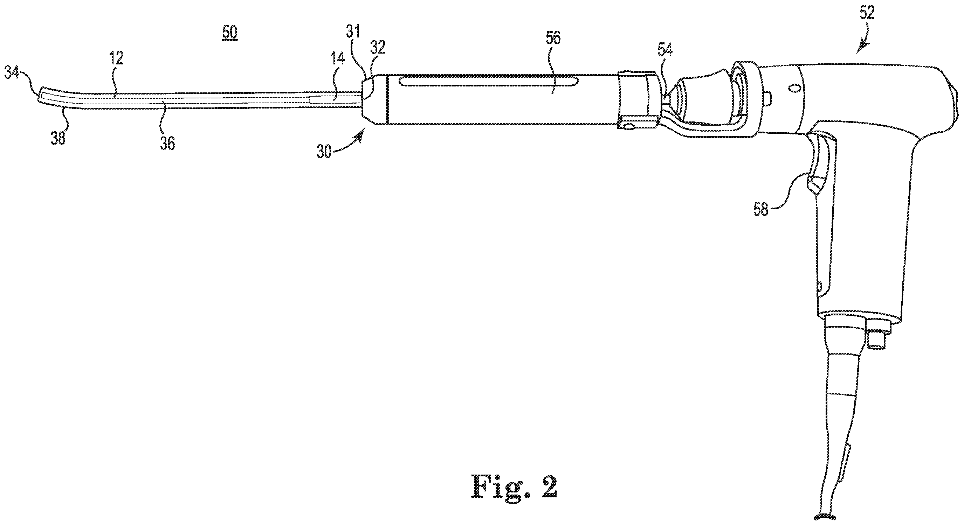

Fig. 2 is a side view of a second embodiment of a delivery device 50 that includes the delivery tube 12 and plunger 14 as discussed above with respect to Fig. 1. As opposed to the hand operated mechanism 18 of device 10, device 50 includes a motorized actuator mechanism 52 that includes a motor to deliver powered rotational movement to a rod 54. A translation mechanism 56 translates the rotational movement of the rod 54 to linear movement so as to move plunger 14 relative to tube 12. In particular, hand piece 52 includes a trigger mechanism 58 that, when depressed, provides rotational power to the rod 54.

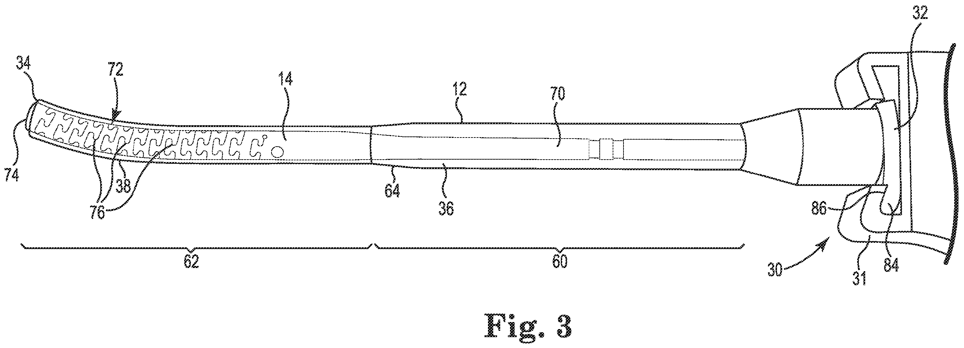

Regardless of a particular actuator mechanism used to position plunger 14 with respect to tube 12, Fig. 3 is a close up view of the tube 12 and plunger 14. As compared with Fig. 1, plunger 14 is illustrated in Fig. 3 in an extended or delivery position, wherein the plunger 14 extends to the distal end 34 of the tube 12. Tube 12 includes a proximal portion 60 and a distal portion 62. The proximal portion 60 extends from the connection mechanism 30 and narrows at a tapered portion 64 to connect with the distal portion 62. Distal portion 62 defines a constant diameter from the tapered portion 64 to the open distal end 34. In one embodiment, an outer diameter of the distal portion is in a range of approximately 5.0 -7.0 millimeters. The tapered portion 64 can assist in preventing material from being compacted within the lumen 36.

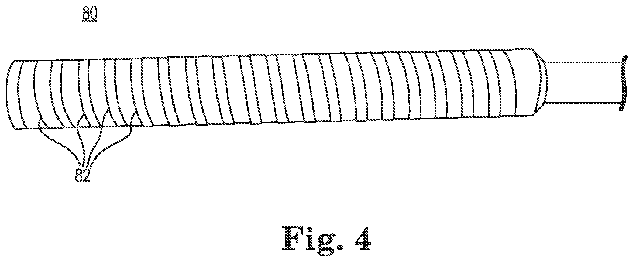

As illustrated, plunger 14 includes a proximal rod 70, a flexible distal portion 72 and a distal tip 74. In one embodiment, the plunger 14 is formed of stainless steel or polyether ether ketone (PEEK). Other materials can be used to form the plunger 14. Prior to use, the plunger 14 can be sterilized. Rod 70 is directly coupled to an actuator mechanism (e.g., mechanism 18 or 52) to move plunger 14 along the tube 12. Flexible distal portion 72 includes a plurality of cuts 76 that impart flexibility within the distal portion 72. In the embodiment illustrated, cuts 76 are dovetail shaped. In alternative embodiments, the cuts 76 can be spiral shaped, double spiral shaped and/or other shapes as desired. For example, Fig. 4 illustrates an alternative plunger 80 that includes double spiral cuts 82 positioned along a length of the plunger 82. Independent of the shape of cuts 76, the cuts 76 impart flexibility on the distal portion 72 so as to allow plunger 14 to move along bend portion 38. Distal tip 74 can be formed of silicon or other surgically safe material as desired.

To secure tube 12 to hand piece 18, the connection mechanism 30 defines a flange 84 positioned at the proximal end 32. Flange 84 is positioned within recesses 86 (one of which is shown) in receiving mechanism 31 of the hand piece 16. To connect connection mechanism 30 to receiving mechanism 31, flange 84 is inserted into receiving mechanism 31 and rotated a quarter turn to position flange 84 within recesses 86.

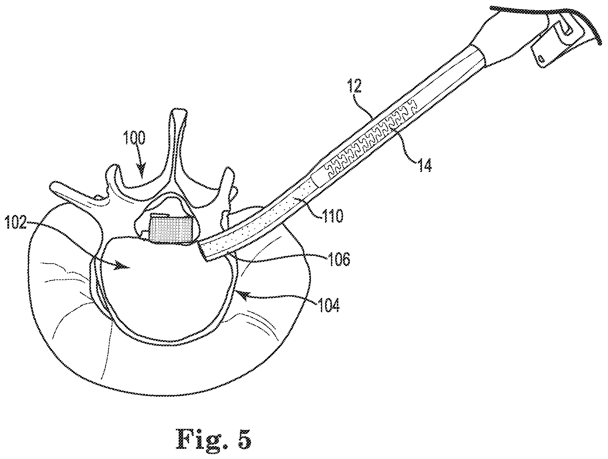

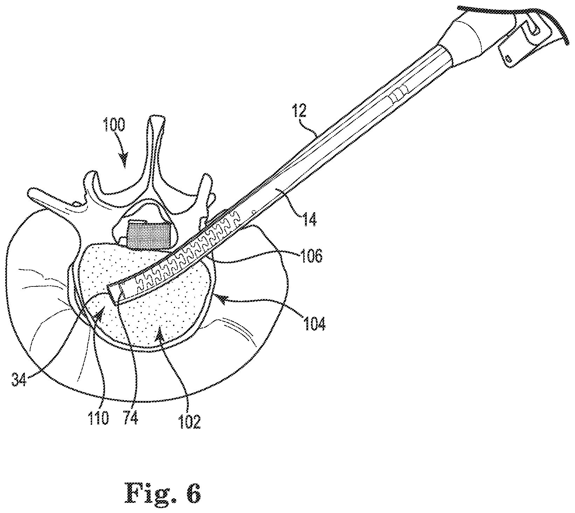

Figs. 5 and 6 illustrate delivery of material to a cavity at a target site. In the embodiment illustrated, the target site is an intervertebral disc 100. Delivery tube 12 can be modified as desired for delivery of graft material to alternative target sites. For example, bend portion 38 can be positioned at different distances from distal end 34 and/or define a different radius of curvature. By way of reference, the intervertebral disc 100 generally includes a nucleus 102 forming a cavity surrounded by an annulus 104. The tube 12 is employed to deliver graft material 110 positioned within lumen 36 to the nucleus 102. In particular, the plunger 14 is in a loading position (i.e., retracted from distal end 34) and graft material 110 has been positioned within lumen 36. Once the material 110 is loaded, tube 12 approaches the intervertebral disc 100. In one embodiment, the tube 12 is deployed using a transforaminal approach, for example during a transforaminal lumbar interbody fusion (TLIF) procedure. Alternative approaches to the disc 100 are also acceptable and include a posterior approach, posterior-lateral approach, anterior approach, left or right lateral approach, etc.

Regardless of the approach, the tube 12 is positioned through an opening 106 formed in the annulus 104. The opening 106 can be a naturally-occurring tear or similar passage. Alternatively, the opening 106 can be surgically cut or otherwise created in the annulus 104. In Fig. 5, the open distal end 34 of the tube 12 is positioned within the nucleus 102 for delivery of graft material therein. In particular, the plunger 14 is in a retracted position, allowing graft material 110 to be positioned within the lumen 36 distal the tip 74. The distal end 34 is advanced into the nucleus 102 to a desired position. Once positioned within the nucleus 102, as illustrated in Fig. 6, the plunger 14 is distally advanced to an extended position to dispense graft material 110 out the distal end 34 of the delivery tube 12.

Although the present disclosure has been described with reference to preferred embodiments, workers skilled in the art will recognize that changes can be made in form and detail without departing from the scope of the present invention as defined by the appended claims.

## Claims

1. A delivery device (10, 50) for delivering graft material to a target site, comprising:
a hand piece (16, 52) maintaining an actuator mechanism (18, 52);
a tube (12) defining a lumen (36), an open end (34) and a connection mechanism (30) coupled to the hand piece, wherein the tube defines a bend portion (38) along its length; and
a plunger (14, 80) positioned within the lumen of the tube and coupled to the actuator mechanism, wherein the plunger includes a flexible distal portion (72) having a plurality of cuts (76, 82), and further wherein a shape of the cuts is one of a spiral cut, a double spiral cut and a dovetail cut;
wherein the actuator mechanism is configured to move the plunger relative to the open end within the lumen from a loading position to an extended position, with the plurality of cuts imparting flexibility in the flexible distal portion such that the plunger moves within the lumen along the bend portion when the actuator moves the plunger from the loading position to the extended position.

2. The device of claim 1, wherein the plunger defines a distal tip (74) formed of silicon.

3. The device of claim 2, wherein the flexible distal portion is formed of stainless steel.

4. The device of claim 2, wherein the flexible distal portion is formed of polyether ether ketone.

5. The device of claim 1, wherein the actuator mechanism includes a ratchet mechanism (26).

6. The device of claim 1, wherein the actuator mechanism includes a motor.

7. The device of claim 1, wherein the tube further defines a proximal portion (60), a distal portion (62), wherein the proximal portion extends from the connection mechanism (30) and narrows at a tapered portion (64) to connect with the distal portion, and wherein the diameter of the tube is constant along the distal portion to the open distal end (34).

## Patentansprüche

1. Abgabevorrichtung (10, 50) zum Abgeben von Pfropfmaterial an einen Zielplatz, umfassend:
ein Handstück (16, 52), das einen Aktuatormechanismus (18, 52) führt;
eine Röhre (12), die ein Lumen (36), ein offenes Ende (34) und einen Verbindungsmechanismus (30), der an das Handstück gekoppelt ist, definiert, wobei die Röhre einen Kurvenabschnitt (38) entlang ihrer Länge definiert; und
einen Plunger bzw. Stempel (14, 80), der innerhalb des Lumens der Röhre angeordnet ist und an den Aktuatormechanismus gekoppelt ist, wobei der Plunger einen flexiblen distalen Abschnitt (72)mit einer Mehrzahl von Schnitten (76, 82) aufweist, und weiter wobei eine Form der Schnitte ein spiralförmiger Schnitt, ein doppelspiralförmiger Schnitt oder ein Schwalbenschwanzschnitt ist;
wobei der Aktuatormechanismus konfiguriert ist, um den Plunger relativ zu dem offenen Ende innerhalb des Lumens von einer Ladeposition zu einer erweiterten Position zu bewegen, wobei die Mehrzahl von Schnitten eine Flexibilität in dem flexiblen distalen Abschnitt derart gewähren, dass der Plunger sich innerhalb des Lumens entlang des Kurvenabschnittes bewegt, wenn der Aktuator den Plunger von der Ladeposition zu der erweiterten Position bewegt.

2. Vorrichtung nach Anspruch 1, wobei der Plunger eine distale Spitze (74) definiert, die aus Silizium ausgebildet ist.

3. Vorrichtung nach Anspruch 2, wobei der flexible distale Abschnitt aus rostfreiem Stahl ausgebildet ist

4. Vorrichtung nach Anspruch 2, wobei der flexible distale Abschnitt aus Polyäther-Äther-Keton ausgebildet ist.

5. Vorrichtung nach Anspruch 1, wobei der Aktuatormechanismus einen Ratschenmechanismus (26) aufweist.

6. Vorrichtung nach Anspruch 1, wobei der Aktuatormechanismus einen Motor aufweist.

7. Vorrichtung nach Anspruch 1, wobei die Röhre weiter einen proximalen Abschnitt (60), einen distalen Abschnitt (62) definiert, wobei der proximale Abschnitt sich von dem Verbindungsmechanismus (30) erstreckt und sich bei einem verjüngten Abschnitt verjüngt, um sich mit dem distalen Abschnitt zu verbinden, und wobei der Durchmesser der Röhre entlang des distalen Abschnittes zu dem offenen distalen Ende (34) konstant ist.

## Revendications

1. Dispositif d'administration (10, 50) pour administrer un greffon dans un site cible, comportant :
une pièce à main (16, 52) qui maintient un mécanisme d'actionneur (18, 52) ;
un tube (12) définissant une lumière (36), une extrémité ouverte (34) et un mécanisme de connexion (30) couplé à la pièce à main, dans lequel le tube définit une partie de courbure (38) le long de sa longueur ; et
un piston (14, 80) positionné à l'intérieur de la lumière du tube et couplé au mécanisme d'actionneur, dans lequel le piston comprend une partie distale flexible (72) ayant une pluralité d'encoches (76, 82), et en outre dans lequel une forme des encoches est l'une d'une encoche en spirale, d'une encoche en double spirale et d'une encoche en queue d'aronde ;
dans lequel le mécanisme d'actionneur est configuré pour déplacer le piston par rapport à l'extrémité d'ouverture à l'intérieur de la lumière à partir d'une position de chargement jusqu'à une position étendue, avec la pluralité d'encoches imprimant une flexibilité dans la partie distale flexible de sorte que le piston se déplace à l'intérieur de la lumière le long de la partie de courbure lorsque l'actionneur déplace le piston depuis la position de chargement jusqu'à la position étendue.

2. Dispositif selon la revendication 1, dans lequel le piston définit un embout distal (114) formé de silicone.

3. Dispositif selon la revendication 2, dans lequel la partie distale flexible est formée d'acier inoxydable.

4. Dispositif selon la revendication 2, dans lequel la partie distale flexible est formée de polyétheréthercétone.

5. Dispositif selon la revendication 1, dans lequel le mécanisme d'actionneur comprend un mécanisme de cliquet (26).

6. Dispositif selon la revendication 1, dans lequel le mécanisme d'actionneur comprend un moteur.

7. Dispositif selon la revendication 1, dans lequel le tube définit en outre une partie proximale (60), une partie distale (62), dans lequel la partie proximale s'étend depuis le mécanisme de connexion (30), et se réduit au niveau d'une partie conique (64) pour se connecter à la partie distale, et
dans lequel le diamètre du tube est constant le long de la partie distale jusqu'à l'extrémité distale ouverte (34).
